# EUROPEAN PATENT SPECIFICATION

(11) **EP 3 230 250 B1**
(45) Date of publication and mention of the grant of the patent: **25.09.2019**
(21) Application number: 15812920.5
(22) Date of filing: 08.12.2015
(51) Int. Cl.: C07C 51/12, C07C 53/08

(54) **USE OF PHOSPHATE SALTS IN THE PRODUCTION OF CARBOXYLIC ACIDS**
VERWENDUNG VON PHOSPHATSALZEN BEI DER HERSTELLUNG VON CARBOXYLSÄUREN
UTILISATION DE SELS DE PHOSPHATE DANS LA PRODUCTION D'ACIDES CARBOXYLIQUES

(30) Priority: 09.12.2014 US 201462089505 P
(43) Date of publication of application: 18.10.2017
(73) Proprietor: LyondellBasell Acetyls, LLC, Houston, TX 77010 (US)
(72) Inventor: HALLINAN, Noel, C., Houston, TX 77010 (US); RAMAGE, David, L., Houston, TX 77010 (US); WHITE, Daniel, F., Houston, TX 77010 (US)
(74) Representative: Sacco, Marco
(86) International application number: PCT/US2015/064394
(87) International publication number: WO 2016/094351

(56) References cited:
- WO-A1-2013/090720
- US-A- 5 144 068
- US-B1- 6 395 928

## Description

### CROSS-REFERENCE TO RELATED APPLICATIONS

This application is filed under the Patent Corporation Treaty, which claims benefit of priority to U.S. Provisional Application No. 62/089,505 filed on December 9, 2014.

### BACKGROUND

### I. Technical Field

The present disclosure relates to the field of chemistry In some embodiments, the present disclosure relates to a carbonylation method. In some embodiments, the present disclosure relates to a lithium iodide, LiI, carbonylation method that includes the addition of a phosphate containing additive to the carbonylation method.

### II. Description of Related Art

The LiI carbonylation process is a widely used industrial process to produce carboxylic acids and esters. In particular, the LiI process is commonly used to produce glacial acetic acid. In 2011, the use of metal co-catalyst to the LiI process to achieve enhanced stability of the catalyst in the carbonylation process was described. Some examples of the metal co-catalysts used in the LiI process include lanthanide metals such as lanthanum, cerium, praseodymium, neodymium, promethium, samarium, europium, gadolinium, terbium, dysprosium, erbium, thulium, ytterbium, or lutetium; transition metals such as zinc, beryllium, indium, or tin; or alkaline earth metals such as strontium and barium. As such, simpler, cost-effective, or more efficacious methods of enhancing the catalyst stability and efficacy are commercially desirable for a more effective carbonylation process. WO 2013/090720 discloses a process for the preparation of acetic acid by carbonylation of methanol in the presence of a rhodium catalyst, MeI, LiI and a co-promotor comprising a dissymmetric phosphonium cation. The latter improves the catalyst stability and/or solubilit. US 6,395,298 describes the production of an organic carboxylic acid by reacting an alcohol with carbon monoxide in the presence of a rhodium catalyst system comprising rhodium and methyl iodide and in the presence of a further co-promotor XnM to improve the catalyst stability and/or solubility.

### SUMMARY

In one aspect, the present disclosure provides a method as described in claim 1
In some embodiments, the metal cation M is a Group 1 or Group 2 metal cation. In some embodiments, the metal cation is a Group 1 metal cation. In some embodiments, the phosphate is Na₂HPO₄. In some embodiments, the method comprises maintaining the amount of phosphate in the reaction mixture at about 0.001 M to about 3.0 M. In some embodiments, the amount of phosphate in the reaction mixture at about 0.01 M to about 1.0 M. In some embodiments, the rhodium compound is [H][Rh(CO)₂I₂]. In some embodiments, the method comprises maintaining the amount of rhodium compound in the reaction mixture at about 50 ppm to about 3000 ppm. In some embodiments, the iodide containing compound is methyl iodide. In some embodiments, the method comprises maintaining the amount of the iodide containing compound in the reaction mixture at about 5 wt% to about 20 wt%. In some embodiments, the iodide containing compound is hydrogen iodide or hydroiodic acid. In some embodiments, the reaction mixture further comprises methyl acetate. In some embodiments, the method comprises maintaining the amount of the methyl acetate in the reaction mixture at about 0.5 wt% to about 30 wt%. The metal iodide salt is LiI. The concentration of lithium iodide salt is higher than 1.0 M. In some embodiments, the reaction mixture further comprises water and the amount of water maintained in the reaction mixture is from about 0.1 wt% to about 20 wt% water. In some embodiments, the method provides an increase in the rate of carbonylation of greater than 5%.

While multiple embodiments are disclosed, still other embodiments will become apparent to those skilled in the art from the following detailed description. As will be apparent, certain embodiments, as disclosed herein, are capable of modifications in various aspects. Accordingly, the drawings and detailed description are to be regarded as illustrative in nature and not restrictive.

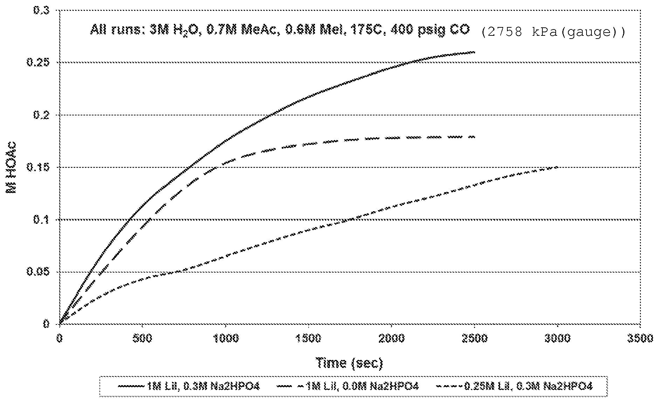

### BRIEF DESCRIPTION OF THE DRAWING

The figure provides a graph of the rate of various LiI carbonylation reaction comprising different concentrations of phosphate containing metal additives.

### DESCRIPTION OF ILLUSTRATIVE EMBODIMENTS

In some aspects, the present disclosure relates to LiI carbonylation process which comprises adding a phosphate containing metal additive. In some embodiments, the present disclosure contemplates a metal phosphoric acid salt which enhances the efficacy of a LiI carbonylation process. The present disclosure contemplates the use of a metal phosphoric acid salt with LiI at certain concentrations to achieve the greatest increase in carbonylation conversion.

### I. Iodide Salt Carbonylation Process

In some aspects, the present disclosure provides a method of carbonylation of methanol to produce a carboxylic acid or ester with the addition of a metal iodide. The metal iodide is LiI. In some aspects, such a LiI carbonylation process as is contemplated by the present disclosure is described in U.S. Patent No. 5,001,259, U.S. Patent No. 5,026,908, U.S. Patent No. 5,144,068, U.S. Patent Application No. 2013/0102809, U.S. Patent Application No. 2013/0102810, U.S. Patent Application No. 2013/0165688, and PCT Publication No. WO 2011/159268.

In some aspects, the present disclosure provides a method of carbonylation which includes the conversion of methanol to acetic acid. In some embodiments, the carbonylation reaction occurs in a liquid reaction medium reactor. In some embodiments, the reactor is a continuous reaction reactor. In some embodiments, the carbonylation reactor is an autoclave which is equipped with a stirring apparatus. In some embodiments of the present disclosure, the reactor comprises mechanism that maintains specific amounts or concentrations of components of the carbonylation reaction. In some embodiments, one of methanol, methyl acetate, or the carbon monoxide is continuously introduced to the reaction chamber. In some embodiments, both methanol or methyl acetate and carbon monoxide are introduced to the reaction chamber. In another embodiment, the carbonylation reaction comprises using a reactor with a flash vessel. In some embodiments, the reactor further comprises a purification section.

In another aspect, the carbonylation method uses a constant pressure of carbon monoxide. In some embodiments, the pressure of carbon monoxide used in the reactor is from about 101 kPa (1 atmosphere) to about 4460 kPa (45 atmospheres) of CO. In some embodiments, the pressure is from about 202 kPa (2 atmospheres) to about 3340 kPa (30 atmospheres) of CO. In some embodiments, the pressure is from about 405 kPa (4 atmospheres) to about 1520 kPa (15 atmospheres). of CO. Additionally, in some embodiments, the absolute pressure of the reactor is higher than the pressure of the carbon monoxide. In some embodiments, the absolute pressure is from about 1520 kPa (15 atmospheres) to 4460 kPa (45 atmospheres). In some embodiments, the absolute pressure comprises the pressure of the methane, carbon monoxide, the pressure of any optional diluent or ballast gases such as hydrogen, carbon dioxide, or an inert gas such as nitrogen or a noble gas, and the pressure of any by-products or the vapor pressure of the liquid components. In some aspects, the carbonylation reaction comprises heating the reaction mixture from about 100 °C to about 350 °C. In some embodiments, the temperature is from about 150 °C to about 250 °C. In some embodiments, the temperature is from about 180 °C to about 220 °C.

In some aspects, the carbonylation reaction comprises a reactor system which allows for the draw off of the liquid reaction components from the reactor and introducing the liquid component into a separation apparatus such that the carbonylation product is removed from the liquid component. In some embodiments, the liquid drawn off from the liquid reaction component is purified to produce the desired carboxylic acid. In some embodiments, the desired carboxylic acid is acetic acid. One non-limiting examples of such a reactor system is described in PCT Publication No. WO 2011/159268. In some embodiments, the rest of the liquid components are re-introduced into the reactor. In other embodiments, the fresh components of the carbonylation reaction are added to the reactor to maintain a specific amount or concentration of the component in the reaction mixture.

In some aspects, the amount of water added to the carbonylation reaction can be used to control the rate of the reaction along with other reaction components. In some embodiments, the carbonylation reaction comprises reacting the carbon monoxide and the alcohol or reactive component with water at a low water concentration. In some embodiments, the low water concentration is less than about 20 wt%. In some embodiments, the low water concentration is less than about 14 wt%. In some embodiments, the low water concentration is less than about 4 wt%. In some embodiments, low water concentration comprises adding water to the reactor from about 0.1 wt% to about 10 wt%. In some embodiments, the low water concentration is from about 0.2 wt% to about 5 wt%. In some embodiments, the low water concentration is from about 0.5 wt% to about 2.5 wt%. In some embodiments, the low water concentration is from about 1.5 wt% to about 2.5 wt%. In other embodiments, the reaction has a high water concentration. In some embodiments, the high water concentration is greater than about 4 wt%. In some embodiments, the high water concentration is greater than about 14 wt%. In general, the carbonylation method when the carbonylation method comprises an iodide salt then the amount of water used is less than 10 wt%. In some embodiments, additional water is formed *in situ* during the reaction process.

In some embodiments, the carbonylation reaction has a liquid reaction medium comprising methyl iodide, methanol or methyl acetate, water, and a carboxylic acid. In some embodiments, the carbonylation reaction comprises adding methyl iodide. In some embodiments, the methyl iodide is added at a concentration from about 2.5 wt% to about 25 wt%. In some embodiments, the concentration of methyl iodide is from about 5 wt% to about 20 wt%. In some embodiments, the concentration of methyl iodide is from 12 wt% to about 16 wt%.

In some embodiments, the carbonylation reaction has a liquid reaction medium comprising an alcohol or reactive component. In some embodiments, the alcohol or reactive component is methanol, dimethyl ether, or methyl acetate. In some embodiments, the carbonylation method uses methyl acetate as the reactive component In some embodiments, the methyl acetate is present in the reaction mixture at a concentration from about 0.5 wt% to about 30 wt%. In some embodiments, the concentration of methyl acetate is from about 0.5 wt% to about 5 wt%. In some embodiments, the concentration is from about 2 wt% to about 5 wt%. In other embodiments, the carbonylation method uses methanol as the reactive component In some embodiments, the carbonylation method further comprises adding methanol to the reaction mixture with methyl acetate.

In another aspect, the present carbonylation method further comprises adding LiI at a concentration higher than 1.0 M. The concentration of lithium iodide which may be used in the carbonylation method varies widely and is dependent on the concentration of the reactive component. Without being bound by theory, the ratio of lithium iodide to methyl acetate used within the carbonylation reaction affects the reaction rate. As described in US Patents 5,001,259, 5,026,908, and 5,144,068, increasing concentrations of lithium iodide and methyl acetate lead to increased rates of reaction. In some embodiment, the lithium to rhodium catalyst is in a molar ratio is greater than 38:1. In some embodiments, the lithium to rhodium catalyst is in a molar ratio is greater than 75:1. In some embodiments, the lithium to rhodium catalyst is in a molar ratio sufficient to stabilize the rhodium catalyst.

In some embodiments, the concentration of iodide is measured by titration of AgNO₃ into a sample of the reaction media and measuring the amount of silver iodide that precipitates from the solution.

In some aspects, the carbonylation method also comprises the desired carboxylic acid. In some embodiments, the desired carboxylic acid is acetic acid. In some embodiments, the balance of the liquid reaction medium is the desired carboxylic acid. In some embodiments, the carboxylic acid is acetic acid. In some embodiments, the liquid reaction medium comprises from about 10 wt% acetic acid to about 95 wt% acetic acid. In some embodiments, the liquid reaction medium comprises a balance of the wt% of acetic acid such that the addition of the amount of the components and the amount of acetic acid is 100 wt%.

In another aspect, the carbonylation method comprises a transition metal catalyst In some embodiments, the catalyst is a rhodium catalyst. Some non-limiting examples of appropriate rhodium carbonylation catalyst include RhCl₃, RhBr₃, RhI₃, RhCl₃•3H₂O, RhBr₃•3H₂O, RhI₃•3H₂O, Rh₂(CO)₄Cl₂, Rh₂(CO)₄Br₂, Rh₂(CO)₄I₂, Rh₂(CO)₈, Rh(CH₃CO₂)₂, Rh(CH₃CO₂)₃, Rh[(C₆H₅)₃P]₂(CO)I, Rh[(C₆H₅)₃P]₂(CO)Cl, elemental Rh, Rh(NO₃)₃, Rh(SnCl₃)[(C₆H₅)P]₂, RhCl(CO)[(C₆H₅)As]₂, RhI(CO)[(C₆H₅)Sb]₂, Rh[(C₆H₅)₃P]₂(CO)Br, Rh[(*n*-C₄H₉)₃P]₂(CO)Br, Rh[(*n*-C₄H₉)₃P]₂(CO)I, RhBr[(C₆H₅)₃P]₃, RhI[(C₆H₅)₃P]₃, RhCl[(C₆H₅)₃P]₃, RhCl[(C₆H₅)₃P]₃H₂, [(C₆Hₛ)₃P]₃Rh(CO)H, Rh₂O₃, [Rh(C₃H₄)₂Cl]₂, K₄Rh₂Cl₂(SnCl₂)₄, K₄Rh₂Br₂(SnBr₂)₄, [H][Rh(CO)₂I₂], K₄Rh₂I₂(SnI₂)₄, or is a complex of the formula [Rh(CO)₂X₂][Y] wherein X is a halide and Y is a proton, an alkali metal cation, or a quaternary compound of nitrogen, phosphorus, or arsenic, or is a similar rhodium complex. In some embodiments, the carbonylation reaction comprises a rhodium catalyst concentration from about 200 ppm to about 1000 ppm. In some embodiments, the rhodium catalyst concentration is from about 300 ppm to about 600 ppm. In some embodiments, the rhodium catalyst concentration is about 400 ppm.

Additionally, in some embodiments, the carbonylation reaction further comprises adding hydroiodic acid. In some embodiments, the hydroiodic acid is added to the reaction mixture to generate *in situ* an iodide salt. In some embodiments, the carbonylation reaction further comprises a method of generating lithium iodide or another iodide salt *in situ.* Some non-limiting methods of *in situ* iodide salt generation are described in US Patent Application 2013/0102809. In some embodiments, other metal salts such as lithium acetate are used as they react with methyl iodide and/or hydroiodic acid to generate anionic iodide. Additionally, the generation of *in situ* iodide, in some embodiments, is from neutral or non-ionic precursors, including, but not limited to, phosphines, amines, amino acids, or other nitrogen or phosphorus containing compounds. Without being bound by theory, the addition of these compounds with methyl iodide or hydroiodic acid results in the generation of anionic iodide through the reaction of methyl iodide with hydroiodic acid. Additionally, the carbonylation reaction, in some embodiments, further comprises a carbonylation catalyst which is a source of iodide. Such carbonylation catalysts are described in Chinese Publication CN1345631 and Chinese Application No. 00124639.9, Chinese Publication CN1105603 and Chinese Application No. 94100505.4, and Chinese Publication CN1349855 and Chinese Application No. 00130033.4.

In some embodiments of the present disclosure, the carbonylation reaction further comprises one or more neutral or inert diluent gases. In some embodiments, the inert gases are noble gases, nitrogen, carbon dioxide, or a hydrocarbon. In some embodiments, the hydrocarbon is a paraffinic hydrocarbon. In some embodiments, the hydrocarbon is a C₁-C₄ hydrocarbon. In some embodiments, the inert gas is nitrogen.

In some embodiments, the carbonylation reaction further comprises one or more metal promoter or co-catalyst compounds which may be used with the present disclosure. In some embodiments, the metal promoter or co-catalyst compound comprises a transition, lanthanide, or actinide metal. In some embodiments, the metal promoter or co-catalyst compound comprises a transition metal selected from chromium, nickel, iron, molybdenum, bismuth, tin, zinc, yttrium, or ruthenium. In some embodiments, the transition metal is a salt such as M(OH)ₓ, M(CO)ₓ, MFₓ, MClₓ, MBrₓ, MIₓ, MOₓ, M(PO₄)ₓ, M(OAc)ₓ, M(NO₃)ₓ, M(CO₃)ₓ, or other commercially available salt. In some embodiments, the metal promoter or co-catalyst is an alkaline earth metal. In some embodiments, the metal is beryllium. In some embodiments, the metal promoter or co-catalyst is a lanthanide metal selected from lanthanum, cerium, praseodymium, neodymium, promethium, samarium, europium, gadolinium, terbium, dysprosium, erbium, thulium, and lutetium. In some embodiments, the metal promoter or co-catalyst is an actinide metal selected from thorium, uranium, or plutonium. In some embodiments, the lanthanide or actinide metal salts are an acetate, a chloride, a iodide, a carbonyl, or a nitrate salt Without being bound by theory, the addition of the metal co-catalyst or promoter enhances the catalytic efficacy of the reaction by preventing the inactivation of the rhodium catalyst such as decreases the extent to which the rhodium catalyst precipitates out of solution.

Additionally, in some embodiments, the metal co-catalyst or promoter is a heteropoly acid. In some embodiments, a heteropoly acid is a complex protic acid which contains at least a hydrogen, a metal, oxygen, and one or more atoms from the p-block such as boron, aluminum, gallium, silicon, phosphorus, arsenic, antimony, iodide, or germanium or a transition metal such as nickel, chromium, tin, or vanadium. In some embodiments, the heteropoly acid is strong Brönsted acids. Without being bound by theory, a heteropoly acid used in the present disclosure is formed by the condensation of two or more inorganic oxyacids with at least one metal and one or more atoms from the p-block or a transition metal. In some embodiments, the heteropoly acid comprises a hetero atom or a central atom with a coordinating element including a poly atom. In some non-limiting examples, the heteropoly acid comprises from about 2 to about 20 poly atoms, oxygen-linked polyvalent metal atoms which surround one or more hetero atoms. In some embodiments, the hetero atom is a p-block atom, a transition metal, a lanthanide, or an actinide. In some embodiments, each hetero atom is independently selected from a copper, beryllium, zinc, nickel, phosphorus, silicon, boron, aluminum, germanium, gallium, iron, cerium, cobalt, arsenic, antimony, bismuth, chromium, tin, titanium, zirconium, vanadium, sulfur, tellurium, manganese, platinum, thorium, hafnium, or iodide. In some embodiments, the poly acid is a transition metal. In some embodiments, each poly atom is independently selected from molybdenum, tungsten, vanadium, chromium, niobium, or tantalum. Some non-limiting examples of hetero poly acids which may be used with the present disclosure include phosphomolybdic acid, tungstosilicic acid, tungstophosphoric acid, molybdosilicic acid, molybdophosphoric acid, molybdotungstophosphoric acid, molybdotungstosilicic acid, vanadotungstophosphoric acid, vanadotungstosilicic acid, vanadomolybdosilicic acid, vanadomolybdophosphoric acid, tungstoboric acid, molybdoboric acid, molybdotungstoboric acid. Additionally, in some embodiments, the heteropoly acid is a Keggin heteropolyanion of the formula: XM₁₂O₄₀^{x-8} wherein X is a p-block atom, M is a metal atom, and x is the oxidation state of the oxidation state of the p-block atom. In some embodiments, the p-block atom is silicon or phosphorus with an oxidation state of +4 and +5, respectfully. In some embodiments, the metal atom is molybdenum or tungsten.

In some embodiments, the hetero poly acid comprises a phosphorus or silicon hetero atom and has at least one poly atom selected from tungsten, molybdenum, chromium, vanadium, and tantalum. In some embodiments, the hetero poly acid has a formula: H₃M₁₂XO₄₀, wherein M is the poly atom and X is the hetero atom. In some embodiments, the poly atom is molybdenum or tungsten.

Without being bound by theory, the use of a metal co-catalyst or promoter increases the catalytic activity of the rhodium catalyst by increasing the solubility of the catalyst in the reaction media. In some embodiments, rhodium solubility is increased in environments which are rich in carbon monoxide as rhodium carbonyl, rhodium iodide, or rhodium carbonyl iodide complexes are generally soluble in acetic acid and/or water. When the environment is depleted of carbon monoxide, the solubility of the rhodium catalyst decreases as the rhodium catalyst composition changes causing the rhodium catalyst to precipitate.

### II. Metal Phosphate Compounds

the phosphate has the formula:

MₓH_{y}PO₄ (I)

wherein: M is a metal cation, x is 0, 1, or 2 , y is 1, 2 or 3.

In some aspects, the amount of the metal phosphate compound added to the reaction comprises a concentration from about 0.01 M to about 10.0 M. In some embodiments, the concentration is from about 0.01 M to about 3.0 M. In some embodiments, the concentration of the metal phosphate compound is from about 0.05 M to about 1.0 M. In some embodiments, the concentration is from about 0.1 M to about 0.3 M. The optimization of a concentration of the metal phosphate compound for a particular reaction system would be obvious to a person of skill in the art Optimization of the concentration of the metal phosphate is modulated based upon the amount of a specific additive to the carbonylation reaction. In some embodiments, the specific additive is lithium iodide. In some embodiments, as the concentration of lithium iodide is varied, the concentration of the metal phosphate compound is changed. Without being bound by theory, under specific reaction conditions, as the concentration of lithium iodide is increased, increasing the concentration of the metal phosphate compound leads to an increase in the carbonylation rate.

In one aspect, the optimization of the concentration of the metal phosphate compound depends on the concentration and reaction conditions of the carbonylation process. In some embodiments, the modulation of the amount of lithium iodide in the reaction affects the amount of the co-catalyst or promoter that is used. The concentration of lithium iodide is greater than 1.0 M. In some embodiments, as the concentration of the lithium iodide is increased up to a concentration of 2.0 M LiI or about 26.67 wt% of LiI, the addition of more of the metal phosphate compound leads to increased activity. In some embodiments, the optimization of the concentration of the metal phosphate compound can be carried out through routine optimization of the carbonylation process when the concentration of the additional components is known. Without being bound by theory, one method of optimizing the concentration of the metal phosphate compound that may be used is a binary search method which comprises testing a concentration on each side of the first concentration and measuring the activity of these two concentrations. If the activity changes, additional concentrations around the higher activity are measured until the optimal activity is identified.

In another aspect of the present disclosure, the efficacy of the addition is measured with changes in the space-time yield (STY). In some embodiments, the STY is defined as g/mol carbonylation product per volume of reaction solution per unit time. In some embodiments, an improved STY is one which is equal to or greater than the same composition without the presence of the additional promoter or co-catalyst. In some embodiments, the same composition comprises using the same amount of rhodium catalyst. In other embodiments, the same composition further comprises using the same concentration of lithium iodide and other additives or co-catalysts.

### III. Process Scale-Up

The above methods can be further modified and optimized for preparative, pilot- or large-scale production, either batch or continuous, using the principles and techniques of process chemistry as applied by a person skilled in the art. Such principles and techniques are taught, for example, in Practical Process Research & Development (2012).

### IV. Definitions

The use of the word "a" or "an," when used in conjunction with the term "comprising" in the claims and/or the specification may mean "one," but it is also consistent with the meaning of "one or more," "at least one," and "one or more than one."

Throughout this application, the term "about" is used to indicate that a value includes the inherent variation of error for the device, the method being employed to determine the value, or the variation that exists among the study subjects.

The terms "comprise," "have" and "include" are open-ended linking verbs. Any forms or tenses of one or more of these verbs, such as "comprises," "comprising," "has," "having," "includes" and "including," are also open-ended. For example, any method that "comprises," "has" or "includes" one or more steps is not limited to possessing only those one or more steps and also covers other unlisted steps.

A "Group 1 metal" or an "alkali metal" comprises an atom or ion selected from the elements: lithium, sodium, potassium, rubidium, cesium, francium, or another element in the first column of the periodic table. In some embodiments, the term is a cation of lithium, sodium, potassium, rubidium, or cesium with a +1 charge. A "Group 2 metal" or an "alkaline earth metal" comprises an atom or ion selected from the elements: beryllium, magnesium, calcium, strontium, barium, or radium. In some embodiments, the term is a cation of beryllium, magnesium, calcium, strontium, or barium with a +2 charge. A "transition metal" comprises an atom or ion selected from an element in Groups 3-12 between scandium and zinc, ytterbium and cadmium, and lutetium and mercury, inclusively, on the periodic table. A "lanthanide" or "lanthanoid" comprises an atom or ion selected from an element between lanthanum and ytterbium, inclusively, on the periodic table. An "actinide" or "actinoid" comprises an atom or ion selected from an element between actinium and nobelium, inclusively, on the periodic table.

An "iodide containing compound" is a compound which contains a highly polarizable bond between an iodide atom and a hydrogen or carbon in which the iodide atom has a formal oxidation state of -1. Some non-limiting examples of an "iodide containing compounds" includes, but is not limited to, hydrogen iodide, hydroiodic acid, methyl iodide, or hexyl iodide.

A "method" is series of one or more steps undertaking lead to a final product, result or outcome. As used herein, the word "method" is used interchangeably with the word "process".

### V. Examples

### Example 1: Carbonylation with Metal Phosphorus Salts

The experiments described below were carried out in batch mode using a 300 mL autoclave constructed of Hastalloy C-276. The reactor head was equipped with attachments for cooling coils, thermocouples and dip tubes for sample exit and return. Loss of vapor to the vapor stack was minimized by two in-series condensers. The reaction components minus the catalyst were charged to the reactor. After leak testing with nitrogen and purging with CO, the reactor and its contents were heated to the desired temperature at a CO pressure of 0,69x10⁶ Pa (gauge) - 1,38x10⁶ Pa (gauge) (100 - 200 psig) with agitation.

The reaction was then started by injecting rhodium-containing catalyst into the reactor and then raising the pressure of the reactor to 2,76x10⁶ Pa (gauge) (400 psig). The reaction was allowed to proceed at constant pressure, which was maintained by feeding CO from a high pressure reservoir via regulator. The extent of the carbonylation reaction was measured by the pressure drop in the reservoir. The pressure drop was converted to moles of CO reacted using the known reservoir volume. At run termination when no further CO uptake was observed, the batch reactor was cooled and a sample removed for gas chromatographic analysis.

Data associated with the seven runs as contained in Table 1 were obtained in which in addition to the components listed in Table 1, the following components were present at the start of every run.
- 3 M H₂O
- 0.6 M MeI
- 0.7 M methyl acetate
- 4.4 mM Rh (added as rhodium acetate)

The following conditions were common to all runs: 175 °C, 2,76 x 10⁶ Pa (gauge) (400 psig). CO, and a total reactor volume of 210 mL's in which bulk solvent was acetic acid.

In Table 1, the column labelled "STY" refers to space-time-yield which has units of moles•L⁻¹•hr⁻¹. This rate measurement is associated with the initial period of the reaction during which component concentrations have not changed substantially from their starting concentrations and in which CO uptake varies linearly with time elapsed. The column labelled "% HOAc Yield" refers to the total amount of acetic formed over the course of the run as measured by CO consumption, as a percentage of the theoretical maximum acetic that could form based on starting methyl acetate concentration.

**Table 1: Carbonylation Results with the Addition of a Metal Phosphate at Different LiI Concentrations (the examples shown in the table below are not according to the invention)**

| Metal Phosphate Concentration (M) | 0.25 M LiI | 0.33 M LiI | 1.0 M LiI |
|---|---|---|---|
| 0.0 M Na₂HPO₄ | 3.42 | 3.43 | 3.46 |
| 0.1 M Na₂HPO₄ | ND | 2.5 | 3.65 |
| 0.3 M Na₂HPO₄ | 1.52 | ND | 4.02 |

| | | | |
|---|---|---|---|
| * ND is not determined. | | | |

As can be seen in Table 1, the addition of a metal phosphate results in a rate increase when added to a reaction with a 1.0 M LiI concentration. At lower concentrations of LiI, the rate of carbonylation was decreased when a metal phosphate was added to the reaction mixture. Similar effects are described in the figure.

### REFERENCES

The following references provide exemplary procedural or other supplementary details.
U.S. Patent No. 5,001,259
U.S. Patent No. 5,026,908
U.S. Patent No. 5,144,068
U.S. Patent Application 2013/0102809
U.S. Patent Application 2013/0102810
U.S. Patent Application 2013/0165688
PCT Publication WO 2011/159268
Chinese Patent Publication CN1345631/Chinese Application No. 00124639.9
Chinese Patent Publication CN1105603/Chinese Application No. 94100505.4
Chinese Patent Publication CN1349855/Chinese Application No. 00130033.4
Anderson, N.G., Practical Process Research & Development - A Guide For Organic Chemists, 2nd ed., Academic Press, New York, 2012.

## Claims

1. A method comprising reacting methanol with carbon monoxide in a reaction mixture in the presence of:
(A) a rhodium compound;
(B) an iodide containing compound;
(C) lithium iodide, LiI at a concentration higher than 1.0 M and
(D) a phosphate of the formula:
MxHyPO4 (I)
wherein:
M is a metal cation;
x is 0, 1, or 2; and
y is an integer equal to 3-x;
under conditions sufficient to cause carbonylation of methanol to form acetic acid.

2. The method of claim 1, wherein the metal cation is a Group 1 or Group 2 metal cation.

3. The method of claim 2, wherein the metal cation is a Group 1 metal cation.

4. The method of claim 3, wherein the phosphate is Na2HPO4.

5. The method of claim 1 comprising maintaining the amount of phosphate in the reaction mixture at about 0.001 M to about 3.0 M.

6. The method of claim 5 comprising maintaining the amount of phosphate in the reaction mixture at about 0.01 M to about 1.0 M.

7. The method of claim 1, wherein the rhodium compound is [H][Rh(CO)2I2].

8. The method of claim 1 comprising maintaining the amount of rhodium compound in the reaction mixture at about 50 ppm to about 3000 ppm.

9. The method of claim 1, wherein the reaction mixture further comprises methyl acetate.

10. The method of claim 12 comprising maintaining the amount of the methyl acetate in the reaction mixture at about 0.5 wt% to about 30 wt%.

## Patentansprüche

1. Verfahren, umfassend Umsetzen von Methanol mit Kohlenmonoxid in einer Reaktionsmischung in Gegenwart von:
(A) einer Rhodiumverbindung;
(B) einer Iodid enthaltenden Verbindung;
(C) Lithiumiodid, LiI, in einer Konzentration von mehr als 1,0 M, und
(D) einem Phosphat mit der Formel:
MₓH_{y}PO₄ (I)
wobei:
M ein Metallkation ist;
x 0, 1 oder 2 ist; und
y eine ganze Zahl gleich 3-x ist,
unter Bedingungen, die ausreichen, um Carbonylierung von Methanol unter Bildung von Essigsäure zu bewirken.

2. Verfahren nach Anspruch 1, wobei das Metallkation ein Metallkation der Gruppe 1 oder Gruppe 2 ist.

3. Verfahren nach Anspruch 2, wobei das Metallkation ein Metallkation der Gruppe 1 ist.

4. Verfahren nach Anspruch 3, wobei das Phosphat Na₂HPO₄ ist.

5. Verfahren nach Anspruch 1, umfassend Halten der Menge an Phosphat in der Reaktionsmischung auf etwa 0,001 M bis etwa 3,0 M.

6. Verfahren nach Anspruch 5, umfassend Halten der Menge an Phosphat in der Reaktionsmischung auf etwa 0,01 M bis etwa 1,0 M.

7. Verfahren nach Anspruch 1, wobei die Rhodiumverbindung [H][Rh(CO)₂I₂] ist.

8. Verfahren nach Anspruch 1, umfassend Halten der Menge an Rhodiumverbindung in der Reaktionsmischung auf etwa 50 ppm bis etwa 3000 ppm.

9. Verfahren nach Anspruch 1, wobei die Reaktionsmischung ferner Methylacetat umfasst.

10. Verfahren nach Anspruch 12, umfassend Halten der Menge an Methylacetat in der Reaktionsmischung auf etwa 0,5 Gew.% bis etwa 30 Gew.%.

## Revendications

1. Procédé comprenant la réaction de méthanol avec du monoxyde de carbone dans un mélange réactionnel en présence :
(A) d'un composé de rhodium ;
(B) d'un composé contenant de l'iodure ;
(C) d'iodure de lithium, LiI en une concentration supérieure à 1,0 M et
(D) d'un phosphate de formule :
MₓH_{y}PO₄ (I)
dans laquelle :
M représente un cation métallique ;
x représente 0, 1 ou 2 ; et
y représente un nombre entier égal à 3-x ;
dans des conditions suffisantes pour provoquer la carbonylation du méthanol pour former de l'acide acétique.

2. Procédé selon la revendication 1, le cation métallique étant un cation métallique du groupe 1 ou du groupe 2.

3. Procédé selon la revendication 2, le cation métallique étant un cation métallique du groupe 1.

4. Procédé selon la revendication 3, le phosphate étant le Na₂HPO₄.

5. Procédé selon la revendication 1, comprenant le maintien de la quantité de phosphate dans le mélange réactionnel à environ 0,001 M jusqu'à environ 3,0 M.

6. Procédé selon la revendication 5, comprenant le maintien de la quantité de phosphate dans le mélange réactionnel à environ 0,01 M jusqu'à environ 1,0 M.

7. Procédé selon la revendication 1, le composé de rhodium étant [H][Rh(CO)₂I₂].

8. Procédé selon la revendication 1, comprenant le maintien de la quantité de composé de rhodium dans le mélange réactionnel à environ 50 ppm jusqu'à environ 3000 ppm.

9. Procédé selon la revendication 1, le mélange réactionnel comprenant en outre de l'acétate de méthyle.

10. Procédé selon la revendication 12, comprenant le maintien de la quantité d'acétate de méthyle dans le mélange réactionnel à environ 0,5% en poids jusqu'à environ 30% en poids.
